# EUROPEAN PATENT SPECIFICATION

(11) **EP 2 023 903 B1**
(45) Date of publication and mention of the grant of the patent: **23.04.2014**
(21) Application number: 07733860.6
(22) Date of filing: 18.05.2007
(51) Int. Cl.: A61K 9/16

(54) **PROCESS FOR THE PREPARATION AND SURFACE COATING OF PELLETS**
VERFAHREN FÜR DIE VORBEREITUNG UND OBERFLÄCHENBESCHICHTUNG VON TABLETTEN
PROCÉDÉ DE PRÉPARATION ET DE REVÊTEMENT DE SURFACE DE GRANULÉS

(30) Priority: 19.05.2006 HU 0600424; 15.05.2007 HU 0700343
(43) Date of publication of application: 18.02.2009
(73) Proprietor: EGIS Gyógyszergyár Nyilvánosan Müködõ Részvénytársaság, 1106 Budapest (HU)
(72) Inventor: FARAGO, Gábor, H-9900 Körmend (HU); FAZEKAS, Patrik, H-9900 Körmend (HU); GÜNTHER, Gábor, H-9900 Körmend (HU); KOCSIS, László, H-9900 Körmend (HU); PATAKI, Károly, H-8200 Veszprém (HU)
(74) Representative: Stolmár & Partner
(86) International application number: PCT/HU2007/000043
(87) International publication number: WO 2007/135470

(56) References cited:
- WO-A-99/42087
- WO-A-2005/034930

## Description

### TECHNICAL FIELD OF THE INVENTION

The present invention relates to process for the preparation of pellets and process for surface-coating thereof. The process according to the present invention is especially suitable for the production of pellets containing a pharmaceutically active ingredient such as potassium chloride and providing pellets prepared according to the present invention with a coating, especially with a coating suitable for the controlled release of said active ingredient.

### TECHNICAL BACKGROUND OF THE INVENTION

The importance of the medicaments containing potassium chloride as active ingredient is increasing, which is demonstrated by the increasing turnover of such medicaments. Such preparations are mostly intended for compensation of the decrease in the concentration of potassium ions in body fluids. For example, during diuretic treatment, the concentration of potassium ions decreases, which has to be resupplied.

Potassium chloride can be efficiently used during the treatment of certain cardiovascular diseases (Pharm. J. 274, 73, 2005; Gyógyszerdészet, 2005. June, p. 399). The potassium supplementation is indispensible in other cases as well, such as during intense sport activity or during the strengthening of the heart musculature. Since the pharmaceutical importance of the medicaments containing potassium, more closely, potassium chloride is great, a continuous need exists for the further development of such medicaments and the processes suitable for the production thereof.

Patients suffering from certain diseases, e.g. from cardiovascular or kidney diseases need to take medicaments for potassium supplementation besides other medicines on the long run, sometimes for their whole life span. Since taking such medicaments is enduring and medication becomes part of the patient's daily routine, an important consideration to be taken into account during the development of such medicines is that said medicine containing potassium ions should be administered infrequently, possibly once a day. At the same time, however, the medicament should provide continuous potassium supplementation.

Such medicaments are retard preparations which release the active ingredient continously in the body in a controlled way. Medicaments suitable for the supplementations of potassium ions presently authorized in Hungary are all such retard preparations with the exception of the preparations intended for the treatment of acute potassium deficiency (Pharmindex Official CD-Rom Database, Issue Quarter 2/2005, National Institute for Pharmacy, Budapest, CMPMedica Információs Kft.: Kalium Durules 1 g tablets, Kalium-R tablets, Kaldyum retard capsules).

In the present description, the expression "potassium" is used interchangeably for the concepts "potassium ion" and "potassium ion concentration" in accordance with the conventions used in the art. In this regard, the expression "potassium" refers to the potassium ion rather than the chemical element potassium. Thus, under the expressions "potassium supplementation", "medicament containing potassium", "potassium deficiency", "potassium concentrations" are meant "potassium ion supplementation", "medicament containing potassium ions", "potassium ion deficiency" and "potassium ion concentration", respectively.

Even after the administration of certain retard tablets, potentially dangerously high local potassium concentrations can be developed. In this regard, the most preferred pharmaceutical dosage form is a pellet.

Pellets are characterized as special granules having an average particle size (expressed as average particle diameter) in the range from a few tenth of millimeter to a few millimeters with small deviation, spheric shape, low surface roughness and the compactness of the particle which is similar to that of extruded particles.

Pellets are usually filled into capsules and administered in this form. The advantageous properties of the pellets are resulting from the above-mentioned properties. Such advantageous properties include:
- The possibility of the preparation of uniform and reproducible coating;
- Controllable release profile of the active ingredient;
- Controllable residence time distribution;
- Low risk of toxicity;
- Excellent patient compliance;
- Possibility of pharmaceutically elegant, appealing presentation.

The possibility of the preparation of uniform and reproducible coating results from the spheroid shape, low surface roughness and high compactness of the pellet particles. The reproducibility of the coating process is the consequence of the similar specific surface area of the subsequent batches, therefore coating processes providing reproducible coating can be developed.

The use of pellets provides for the controlled release of the active ingredient contained therein (in the present case, the potassium ion). Pellets can be provided with a coating having specific thickness, quality and composition, which allows the controlled administration of a greater initial dose and a smaller sustaining dose.

The above-mentioned controlled release of the active ingredient can be achieved by filling the mixture comprising two different types of pellets into a capsule. The first type of pellets is suitable for the fast release of a large dose of the active ingredient, which is achieved by applying a coating on the pellets which is subject to fast dissolution after administration. Thus the active ingredient included in such pellets is released rapidly resulting in an instantenous large dose. The second type of the pellets included in the mixture has a slowly dissolving coating, which results in slow, controlled release of the active ingredient in the body.

In this manner, the optimum plasma concentration specific to the disease treated can be achieved and the adverse effects resulting from the peak concentrations of the active ingredient can be prevented.

An important requirement for achieving the desired therapeutic effect is that the pharmaceutically active ingredient should be present in the specified location in the body for an appropriate period of time. Therefore the residence time distribution of the active ingredient (the property describing the residence time of the active ingredient in specific parts of the gastrointestinal tract) plays important role in the development of the therapeutic effect and in determining the applicable dose.

The use of pellets provides for the control of the residence time distribution. By applying an appropriate coating soluble only at the chemical environment characteristic to specific parts of the gastrointestinal tract, the active ingredient is released only at the specific part having appropriate chemical environment for the dissolution of the coating of the pellets having said specific form of coating.

The use of pellets results in the decrease of toxicity risks. In the case of a retard tablet having surface damage, a large or even toxic dose of the active ingredient can be released in the body. In the case of pellets, a few particles having surface damage release only an insignificant amount of the active ingredient from the toxicology point of view, therefore the increase in the plasma concentration of the active ingredient is also insignificant

From the point of view of the patients, it is important to provide medicaments suitable for convenient administration. In the case when the administration or ingestion of a capsule containing pellets is not possible, the pellets can be administered by opening the capsule and mixing the contents thereof with drink or food.

In the case of medicaments taken for a long period of time, it is important to present said medicaments in a pharmaceutically esthetic, elegant form appealing to the patients, which is easily achieved by using pellets. Pellets can be filled into transparent capsules resulting in appealing medicaments. The esthetic presentation can be further enhanced by using pellets in different colors. A possible method for the presentation of such medicament employs a transparent capsule and different colorings for the pellets having different release profile.

Pellets are used in several branches of the industry including pharmaceuticals, chemicals, agrochemicals, food technology, laundry detergents or even during the processing of iron ore.

Pellets used in the pharmaceutical industry are free flowing, spherical particles having particle size between 0.5-2.0 mm with narrow particle size distribution and a porosity of approximately 10%. These properties are resulting from the need of the pharmaceutical industry and the use of pellets having properties different from the previously mentioned in medicaments is also possible.

There are several processes and equipment suitable for the production of pellets useful in the pharmaceutical technology known according to the state of the art. (for example, see: Isaac Ghebre-Sellassie: Pharmaceutical Pelletization Technology, Marcel Dekker, Inc., New York, Basel, 1989).

The first process developed for the production of pellets is the so-called layering method. In this process, spherical pellets of very good quality can be prepared by providing a core progressively by several layers of coating. If desired, the pellets prepared in this manner can contain several layers. The core usually comprises an inert material, e.g. sugar, starch, sodium chloride particles or the mixture thereof.

The core can comprise granules of the substance or the mixture to be formulated obtained by fractional sieving in order to achieve narrow particle size distribution.

The process of formulation is performed in coating drums or in a special rotating drum prepared for the purpose of pellet production. Such drums comprise three sections of a truncated cone- cylindrical-truncated cone shape.

During the process of pelletization, the appropriately conditioned cores are put into rolling motion by rotating the pelleting drum and are kept in such motion, while the surface of the cores is wetted evenly with a liquid optionally containing a binding agent until the limit of aggregation.

Subsequently the rolling mass (the expression of polling bed" is also used in the art) is dosed evenly with a coating powder using a suitable device, e.g. a vibrating screen until the particles comprising the rolling mass bind the coating powder steadily on their surface. The wetting and coating steps are repeated cyclically several times until the desired shape and size is achieved.

The final shape of the pellets depends on the shape of the cores used as initial particles. According to the process, layered pellets can also be prepared using different wetting liquid and/or coating powder composition in each layering cycle. When the desired size and shape are achieved, the particles are dried. The drying step can be performed in a different equipment but also in the drum wherein the wetting and coating steps took place provided that said drum is suitable for drying.

The pharmaceutically active ingredient can be present in the initial cores , in the coating powder or in the wetting liquid as well.

The disadvantage of the above-mentioned layering process resides in the fact that due to the small shearing force exerted on the surface of the pellets, the surface smoothness of the resulting pellets is sometimes unsatisfactory. As a consequence, the pellets can be bound to each other, which results in high proportion of waste. A further disadvantage is that the processing time is long.

The process finds limited applicability in case of materials having high plasticity. During the process, the proportion of the pellets having a size outside the desired range is high.

Despite of its disadvantages, the process is still used in the pharmaceutical industry. Particles suitable as cores are commercially available. Such particles are called "nonpareil" in the art (Drug Development and Industrial Pharmacy, 27(5), 419-430, 2001).

The second, more important process suitable for the production of pellets is the so-called extrusion-spheronisation process (Isaac Ghebre-Selassie és Charles Martin: Pharmaceutical Extrusion Technology, Marcel Dekker, Inc. 2003). In this process, the appropriately homogenized powder mixture is mixed with a liquid and kneaded until a homogeneous wet mass is obtained, the thus obtained mass is extruded and the extrudate resulting from the extrusion process is spheronized (brought into a spheroid shape). The thus obtained raw pellets are dried. The quality of the pellets is principally determined by the process variables of the extrusion and spheronization.

The extrusion-spheronization process is usually performed in a cochlear extruder, which is capable for compacting the wet mass prepared as described hereinbefore, and the parameters, i.e. pitch ratio, profile, size and shape of breaking plates can be easily adapted according to the requirements of the mixture to be extruded. The compacted wet mass leaves the equipment across the perforated place attached to the end of the equipment. The diameter of the fibers leaving the extruder (the extrudate) is approximately 1 mm. The fiber can be chopped using blades of knives, or in case of a mixture having suitable composition, the fibers break into pieces of the appropriate size by themselves upon leaving the extruder. An important requirement set for the extruder is that the rotation speed of the equipment shall be adjustable, since this process parameter greatly influences the quality of the extrudate.

The spheronizer (marumizer) comprises a disc rotating around a vertical axle and the surrounding cylindrical barrel with a dust-extracting attachment. The gap between the two elements is a few tenth of millimeter. The disc has protrusions and recesses. There are several discs with different surface finish available, since the surface quality of said discs plays important role during the spheronization and compacting processes. The principle of the operation of the device is based on the phenomenon of "mass-stroke". (Pataki, K.: Gördülőréteges-porlasztásos granulálás, Kandidátusi értekezés, MTA Műszaki Kémiai Kutató Intézet, Veszprém, 1989), which is the spheronizing effect resulting from the collision of the particles and the rough disc surface. Besides the surface quality of the spheronizing disc, the rotation speed of the disc also plays an important role in the process.

The final step of the extrusion-spheronization process is the drying, which can be performed in a fluidized-bed equipment or in a disc dryer. The selection of the appropriate drying apparatus is important since the final strength of the pellets depends upon the speed of the drying.

The separation of the pellets having the desired particle size can be performed before or after drying. The separation performed before drying is advantageous although less easily feasible, since the undried pellet particles of smaller particle size can be recycled into the manufacturing process therefore the waste ratio is not increased.

During the spheronization process, the particles are distributed vertically in the device by their particle size in such a manner that the particles of greater size are present in the upper portion of the equipment, while those of smaller size are residing in the lower part of the device. During the manufacturing process, the greater is the particle size, the higher is the position of the particle in the spheronization equipment. The equipment is overloaded, thus - in case of appropriate setting of the process parameters - the pellets of the desired size leave the device thorough the top opening.

Such extrusion-spheronization process is disclosed in European Patent No. 1 252 886 and in the papers mentioned below (Drug Development and Industrial Pharmacy, 27(5), 381-391 (2001); Drug Development and Industrial Pharmacy, 28(4), 451-456 (2002); Pharmaceutical Technology, February 2002, 26-34.).

According to the state of the art, an equipment has been disclosed which is essentially an improved spheronizer with the modifications allowing that all the production processes can take place in a single apparatus. This equipment is essentially a spheronization device suitable for the introduction of a large volume of warm air, spraying of liquids and dosing of powders. Such instrument is produced by Freund Industrial Co., Ltd. Tokio, Japan. According to our knowledge, this type of the equipment has not been introduced widely.

Successful pellet-producing devices are the roto-fluidization equipment operated in batch mode, which are very similar to the above-mentioned spheronizer regarding their construction. Such devices are provided with a variable-speed rotating disc, which provides for ordered particle movement and greater shear force. Furthermore, due to the adjustable gap between the disc and the wall of the equipment, air introduced into the device has greater role in the formation of layer movement and in performing the drying step. These devices preferably combine the phenomenon of fluidization with the application of centrifugal force. During the operation of the equipment, the pellet mass being present present reaches a torus shape wherein the individual particles move around in a circular-spiral course. Such devices have the advantage that all the steps of the pellet production as well as pellet coating can be performed in a single equipment (Hungarian Patent No. 196 717, AAPS PharmSciTech 2000, 1(4) art. 35; Drug Development and Industrial Pharmacy 28(10), p. 1201-1212, 2002).

There are further known processes suitable for the production of pellets (pelletization) according to the state of the art. However, none of these processes have been applied on an industrial scale. Examples for these processes are the pelletization and pellet processing using a modified kneading machine (e.g. J. S. Ramaker: Fundamentals of the high-shear pelletisation process, Groningen, The Netherlands, 2001, ISBN: 90-367-1392-7; A. Dévay, Sz. Pál, I. Antal: European Journal of Pharmaceutical Sciences, 25(1), May 2005. 22-25), or pelletization in the liquid phase, which was called earlier sphere-agglomeration. (e.g. Drug Development and Industrial Pharmacy, 26(11), 1151-1158, 2000). Pelletization based on the formation of a melt has also been disclosed in the art >Schaefer, T., PhD Thesis, Melt agglomeration with polyethylene glycols in high shear mixers, Dept. of Pharmacy, The Royal Danish School of Pharmacy, Denmark, 1996; J. Vincent: Thermoplastic pelletizing with a high-shear granulator, Glatt International Times, No. 18., October 2004, 12-14.).

Pellets are usually provided with a coating for coloring, protection against environmental factors, e.g. heat or light. Pellets provided with appropriate coating are also suitable for producing controlled-release pharmaceutical dosage forms.

The development of a coating system as well as the coating process itself requires great carefulness, especially in the production of coatings intended for the use in controlled-release products. (e.g. C. Graham: Pharmaceutical Coating Technology, Taylor & Francis Ltd., 1995).

During the development and optimization of the coating system, special attention is paid to the type and relative amount of the auxiliary agents capable to modify the properties of the basic coating, the investment and process costs and to the environmental aspects. Due to these concerns, the use of aqueous dispersions is favoured in most cases (James W. McGinity: Aqueous Polymeric Coatings for Pharmaceutical Dosage Forms, Marcel Dekker, 1989; Gy. Buchholz, I. Erős, K. Hódi: Gyógyszer-filmbevonó anyagok és eljárások, Fókuszban az Eudragit®, Gyógyszerészet, November 2004. 651-658.; Nasser Nyamweya-Stephen W., Hoag-Ketan A. Mehta: Film Coating with Aqueous Latex Dispersions, Pharmaceutical Technology Yearbook 2001, 8-26.; M. R. Harris, I. Gherbe - Sellassie, R U. Nesbitt: Water-based coating process for sustained release, Pharmaceutical Technology, 102-107, 10, 1986; United States Patent No. 4786508).

Several processes applicable to the coating of pellets are known from the prior art (K. Pataki, E. Horváth, Z. Ormós: Szemcsék bevonása I., Magyar Kémikusok Lapja, 35(1), 32-38, 1980). During the coating process, provisions for the intense agitation of the particles are required. Furthermore, optimization of the method for the building-up of the coating is necessary.

The intense agitation of the particles can be carried out by the following methods:
- by applying mechanical energy (in rolling-bedr equipment, e.g. coating drums),
- by applying the kinetic energy of a flowing gas (fluidized or geyser-bed, spraying equipment, e.g. "Wurster" types devices (Wurster, D. E. (1953): United States Patent No. 2648609; Wurster, D. E.: J. Am. Pharm. Ass. Sci. Ed. 48, 451, 1959);
- by combining the above-mentioned two methods in a roto-fluidization equipment (e.g. Glatt GPCG 120-plus equipment, Glatt Maschinen- & Apparatebau AG, Pratteln, Switzerland).

In the process of coating solid particulate matter (e.g. pellets), presently the rolling-bed method is the most widely used. In this process, the mechanical energy is transferred to the particles to be coated by a vessel rotating around a horizontal or angularly declined axis. The rotating vessel is usually a rotating drum, a rotating pan or a rotating plate. The speed and direction of the particle movement is determined by the resultant force originating from gravitational force, centrifugal force, friction coefficient between the wall of the device and the particles, the intrinsic friction of the particle mass and the ratio of the above-mentioned forces. Under the balanced effect of the above-mentioned physical forces, the particle mass forms a rolling bed.

During the operation of the coating drums, it should be assured as much as possible that the device be operated as a mixer without dead spots (Rácz-Selmeczi: Gyógyszertechnológia 2., Művelettan-eljárástan, Medicina, Budapest, 2001). Such dead spots of the drum if present can lead to the uneven formation of the coating, mass fluctuations and differences in the size of the particles. The formation of dead volume can be prevented by building deflector plates in, which results in more even movement and thus consistent coating thickness.

The advantages of the rolling-layer coating process are the simplicity of the equipment and the smooth and gentle particle agitation. The most important properties of the process are the following (C. Graham: Pharmaceutical Coating Technology, Taylor & Francis Ltd. 1995.):
- Application of a horizontal, cylindrical rotating drum having perforation on its horizontal barrel;
- conical ending of the drum, which promotes the lateral mixing of the particles by turning them back to the central cylindrical part;
- the homogenization is enhanced by distributing/mixing blades/plates;
- The drying air is introduced into the drum through the lateral perforation and the suction ventillator enclosed into the exit tube attached to a pressure vent below the bed provides suction for the air across the bed in such a way that the exit of the pressure vent is tightly engaged against the outer part of the perforated, bent surface of the drum.

The batch size depends on the bulk density of the particles to be coated. The maximum load can be achieved either in case of high density particles or in case of particles having high packing density and small size.

The minimum load is determined empirically and usually depends upon the size and shape of the particles. In the case when the batch size is smaller than the minimum load, the mixing element of the device can extend beyond the particle bed resulting in failure of the operation or in poor quality coating. Furthermore, the exit of the leaving drying air remains partly uncovered by the particles, thereby the drying air can leave the device without having contact with the particles, which results in poor drying efficiency.

The drying air is usually forwarded through the particle bed by a suction ventilator and is removed from the device through a pipeline. The optimum flow rate of the drying air is usually adjusted according to the desired evaporation rate. The pressure drop of the drying air depends on the pressure drop caused by the particle bed, by the pipeline and by installed auxiliary devices, e.g. filters, solvent recovery system etc.

Since the type of coating, the batch size and the properties of drying air are variable, usually adjustable drying systems are employed. It is also necessary to prevent air pollution. The filters suitable for the prevention of environmental harms increase the pressure drop, thereby they can influence the rate of coating, the evaporation rate and the spraying rate as well.

For the purpose of drying air, either the indoor air of the plant or outdoor air can be used. Drying air needs preconditioning before the use in the drying process, which include the removal of impurities and control of the temperature and humidity thereof. Care should be taken to provide that only preconditioned air should contact the particles under production.

The Pellegrini-type coating equipment employs a different principle wherein the so-called Pellegrini-vessel (producer IMA GS Technology, Bologna, Italy) is applied. The vessels having capacity between 10 - 1000 liters use distributor plates for directing the air instead of perforation. In this way, an even, smooth distrubution of the drying air among the pellets is ensured. The advantage of the Pellegrini-type coating equipment resides in the fact that it can be used for the coating of particles within a wide particle size range, even particles having a particle diameter below 1 mm can be coated using this type of equipment. According to the present knowledge of the inventors, the most consistent coating can be prepared using Pellegrini-type vessels. For the applications where there is no sugar present in the coating mixture, the equipment contains an alternating dosing pump, which is provided with variable speed adjustment, which is automatically controlled by the temperature of the particle bed.

Due to the distribution plates which determine the air flow direction, the device can be operated with a low pressure drop, which in turn decreases the wear of the particles. Such machines are especially suited for the coating of pellets, even for the production of pellets having multiple coatings (i.e. build-up pellets) (Nastruzzi C., Cortesi R., Spadoni A. Vechio C.: Influence of formulation and process parameters on pellet production by powder layering technique, AAps PharmSciTech, 2000, 1(2)).

According to the state of the art, there are several known processes suitable for the preparation of a surface coating on pellets containing potassium chloride.

In Hungarian Patent No. 191102 and Published International Patent Application WO86/04817, a process is disclosed for the coating of coarse crystalline potassium chloride particles employing fluidization, wherein the salt crystals are provided with a coating delaying dissolution. Since the specific surface are of potassium chloride is about 6 m²/kg, a large, approximately 10-30 percent by weight amount of coating material should be applied onto the particles in order to achieve the desired dissolution rate (see also: EP0052075). Therefore the process is complicated and expensive.

United States Patent No. 3415758 discloses the microcapsulation of potassium chloride crystals with ethylcellulose in cyclohexane solvent.

European Patent Application No. 52075 discloses the use of the mixture of ethylacrylate-methylmethacrylate copolymer and ethylcellulose in the form of aqueous dispersion in the ratio of 2.5:1 - 5:1 for the coating of potassium chloride. In this case, similarly to the previous state of the art, a large, approximately 25 percent by weight amount of the coating agent was used, therefore this process is considered to be expensive as well.

In Hungarian Patent No. 221 435, a coating process is disclosed for the preparation of coated potassium chloride containing pellets. According to this disclosure, in this process the use of fluidization or roto-fluidization equipment is preferred.

It can be concluded that there is no known process according to the state of the art wherein the coating of potassium chloride containing pellets is performed in a rolling-bed equipment.

From WO 99/42087 optionally coated pellets of KCl the pellets having been used as seed pellets have been known. However, they are not further processed and used as seed pellets to produce larger particles.

### SUMMARY OF THE INVENTION

The objective of our development work was to provide a process for the preparation of pellets containing potassium chloride, wherein the amount of the waste pellets is significantly decreased.

During the production of pellets using rotofluidization, pellets of different size are obtained, which are subsequently fractionated (e.g. by fractionated sieving) in order to provide a homogeneous particle mass with regard to its particle size for the further production stages. During the fractionation process, the proportion of the fraction having particle size smaller than desired is approximately 20 percent by weight, while 60 percent by weight of pellets having the desired particle size and approximately 20 percent by weight of oversized pellet fraction is obtained. The objective of the present invention is therefore to provide a method for increasing the product fraction during the manufacture of pellets.

The second objective of the present development work was to provide a process suitable for the coating of pellets containing potassium chloride, which results in good quality product and allows the production of coated pellets at a lower cost than in conventional processes.

The present invention is based on the surprising recognition that in the case when the pellet fraction having smaller size than the product fraction is introduced repeatedly into a rotofluidization equipment, said undersized pellets can serve as so-called seeding pellets. Thus the fraction of the pellets having smaller particle size than desired can be utilized as starting seed material, thereby improving the proportion of the product fraction, thus the yield of the process. Furthermore, said undersized fraction of the pellets does not become waste, thereby improving the feasibility and economy of the production and satisfying environmental requirements. Further improvements residing in the recycling of the pellet fraction having smaller particle size than the specification are the following:
- separation of agglomerated particles;
- removing dust bound to the wall of the equipment;
- improving the smooth agitation of the particles produced.

The second surprising recognition serving as a basis of the present invention is that the coating of potassium chloride active ingredient with a large quantity of polymer providing controlled release can be more advantageously performed in a rolling-bed device having unperforated walls than by fluidization technology and said process results in good quality coated product at low cost.

### DESCRIPTION OF THE INVENTION

According the first aspect of the present invention, there is provided a process for the preparation of pellets, which comprises homogenizing potanium chloride or a premix containing thereof with auxiliary agents to obtain a starting powder mixture, spraying the thus obtained powder mixture with a granulating liquid with agitation, spheronizing, drying and fractionating the particles resulting from the process, wherein the starting or the partially pelletized powder mixture already containing pellets is blended with seeding pellets having identical or essentially identical composition with the pellets to be produced but having smaller particle size than the product fraction of said pellets.

According to a preferable embodiment of the invention, the weight proportion of the seeding pellets blended with the starting or partially pelletized powder mixture already containing pellets is between 5 percent by weight and 25 percent by weight, preferably between 15 percent by weight and 20 percent by weight, related to the weight of the starting or partially pelletized powder mixture.

According to another preferable embodiment, the average particle size of the seeding pellets is smaller than the lower particle size limit of the product fraction.

According to a still further preferable embodiment of the invention, the upper particle size limit of the seeding pellets is lower than the lower particle size limit of the product fraction.

In an advantageous embodiment of the invention, the pelletization is carried out as a batch process using rotofluidization technology.

In a still further preferable embodiment of the invention, a pellet fraction having smaller particle size than the product fraction of a production batch is used as seeding pellets.

According to a further preferable embodiment of the invention, the seeding pellets are produced in an independent production stage separated from the pelletization process.

Another aspect of the present invention is related to the coating of potassium chloride containing pellets with a surface coating, characterized in that the coating process is carried out in a rolling-bed equipment having unperforated wall, preferably in a Pellegrini-type vessel.

The prior art is silent about the production and use of seeding pellets.

The concept of "seeding pellet" during the pelletization process is meant analogously to the concept of "seeding crystal" during a crystallization process.

In the present description, the expressions "pellet", "pellets", "seeding pellet", "seeding pellets" and "pellet mass" are understood as a plurality of particles constituting a bulk pellet mass except when explicit reference is made to an individual particle.

The expressions "pelletization" and "pellet production" are used interchangeably and refer to an industrial process of the production or manufacturing pellets.

According to the present invention, any pellet mass can be used as seeding pellets provided that the average particle size thereof is smaller than the average particle size of the the product fraction, therefore it is possible that there exists an overlapping between the particle size range of the product fraction and the seeding pellets.

Preferably, pellets having smaller average particle size than the particle size range of the product fraction can be used as seeding pellets. Especially advantageously, the particle size of the seeding pellets is lower than the lower limit of the particle size range of the product fraction.

For example, in the case when the desired particle size range of the product fraction is between 0.8 - 1.6 mm, then the particle size of the seeding pellets is lower than about 1.2 mm, said particle size is preferably lower than about 0.8 mm, especially preferably the particle size is approximately 0.1 - 0.8 mm.

The seeding pellets having smaller particle size than the average particle size of the product fraction, preferably of particle size which is lower than the lower particle size limit of product fraction are applied by introducing the seeding pellets obtained from an earlier production process to a subsequent production process by mixing with the starting materials or charging continously or in portions during the pelletization.

In the case when the weight of the seeding pellets obtained in a production process is not sufficient to the amount required for the subsequent batch, seeding pellets can be produced for this purpose separately. Thus seeding pellets can be obtained either by the separation of a pellet fraction of small particle size from a production batch or by producing small particle size pellets in an independent process for this purpose separately. Seeding pellets produced by the above-mentioned two methods can be applied separately or in a mixture form.

The use of seeding pellets has several advantages.

One advantage of the use of seeding pellets is that the particle fraction having smaller particle size than desired can be recycled, therefore no waste is produced and the costs of the production including material cost, energy cost, labour cost can be reduced and no environmental harms occur.

A further advantage of the use of seeding pellets resides in the fact that particles introduced into a rotofluidization equipment decrease the possibility of non-desired sticking or aggregation.

According to the present industrial practice, the starting material used in the rotofluidization equipment consists of the powder mixture of the pharmaceutically active ingredient and the auxiliary agents: During the pelletization, the particles often stick together forming aggregates, which prevents the smooth and even movement, thereby the consistent wetting of the particles.

According to the present invention, seeding pellets are added to the starting powder mixture. In this case, the above-mentioned non-desired effect is prevented since the cohesive energy between powder particles resulting in sticking is advantageously compensated by the kinetic energy of the moving seeding pellet particles. In other words, the seeding pellet particles having greater size and higher compactness behave as the balls in the ball mill and separate the aggregated powder particles during a series of collisions.

During the application of the pelletization technology according to the state of the art, a significant amount of powder remains adhered to the wall of the equipment. The amount of the powder thus removed from the pelletization process result in a decrease of the yield. A further advantageous effect of using seeding pellets resides in the fact that the kinetic energy transferred by the seeding pellet abrades the portion of the powder adhered to the wall of the vessel, thereby said portion can be transferred back into the pelletization process.

In the rotofluidization device, seeding pellets perform uniform movement whereby powder particles are also forced to follow a uniform path. Due to the above-mentioned uniform movement, particles pass by the spraying nozzles with the same probability, therefore the probability of being contacted with the wetting-binding agent is identical. Having identical probability of contact, the moistening of the particles becomes uniform, the chance for inappropriately low or high moistening decreases significantly. During the pelletization process, pellets having inappropriately high moisture content become to particles exceeding the desired pellet size, while those wherein the moistening is too low are responsible for the formation of undersized particles. In the case when seeding pellets are used, the pellets produced from the powder particles become more uniform and spherical than in the case when no seeding pellets are employed.

In summary, during the pelletization using seeding pellets, the powder particles do not agglomerate suddenly, do not adhere to the wall of the equipment and the particles perform a smooth, steady, uniform movement, resulting in higher proportion of product fraction and lower amount of waste as compared to the processes known from the art.

During the process of pelletization, the seeding pellets themselves change, since the powder is layering continously onto the surface of seeding pellets, resulting in the increase in the size of the seeding pellets and finally reaching the size range of the product fraction. In this way, the seeding pellets themselves are transformed into pellet product having identical composition and physical qualities with the pellets directly formed from the powder mixture and the wetting liquid.

As it has been mentioned above, seeding pellets can be prepared in two ways.

According to the first process, such a pelletization is performed wherein the wetting periods are shortened, therefore the pellets formed as main fraction in the process have smaller particle size than the product fraction. Pellets thus obtained can be used as seeding pellets.

According to the second process, the pellet fraction having smaller particle size than the product fraction is isolated by fractionating the product of a normal pellet production process and selecting the undersized fraction.

As it has been mentioned before, the present invention is based on the observation that the pellet fraction having smaller particle size than desired can be recycled with favourable results. However, by using this process it is not always possible to produce seeding pellets in a satisfactory amount therefore this amount can be supplemented by seeding pellets prepared specifically for this purpose. Thus, the combination of the two processes can be used most advantageously in the practice.

As it has been described above, it was observed that the production of retard potassium chloride pellets coated with a controlled release coating can be performed in a rolling-bed apparatus as well.

In the processes according to the state of the art, the production of the retard potassium chloride-containing pellets and the coating thereof are carried out in the same apparatus. In the first step, a large batch of bulk pellet mass that may even amount to several tons is prepared as a product fraction having predetermined particle size distribution. Subsequently in the second step using the same apparatus, the surface of the particles is provided with a polymer coating suitable for controlled release of the active ingredient. However, the treatment of the particles in the same apparatus is cumbersome, therefore according to the present invention, the production and coating of the pellets are carried out in separate devices.

For example, in the case of the coating of retard potassium chloride pellets, the aqueous dispersion of the acrylic acid-methylmethacrylate 2:1 copolymer is a suitable polymer for coating. Such a coating system is available commercially from Degussa-Hüls Group under the trade names Eudragit NE 30D and Eudragit NE 40D. The adherence to the predetermined process parameters during the coating process is very important even more because the polymer dispersion contains an emulgeating agent susceptible to crystallization (in the case of Eudragit, the emulgeating agent is 1.5 percent by by weight Nonoxynol 100). Crystallization of the above-mentioned agent in the coating can result in altered active ingredient release profile (Schreder S., Lee, G.: The complex effects of surfactant on drug release from thin films. Proceed. Interm. Symp. Control. Rel. Bioact. Mater. 21, 678-679, 1994; Schreder, S., Lee, G.: Plastifying effect of surfactants - decisive for drug release. Proceed. Interm. Symp. Control. Rel. Bioact. Mater. 22, 398-399, 1995).

During the experiments designed for the optimization of the production of retard potassium chloride pellets, it has been established that production of such pellets in a rolling-bed process results in an increase of the efficiency and the yield and a decrease in the costs.

The quality parameters of retard potassium chloride-containg products is essentially identical to those obtained by the more complicated coating processes known from the art (Hungarian Patent No. 221435). Under "quality parameters" dissolution profile (i.e. the function of the weight or weight proportion of the dissolved active ingredient as the function of time), the surface and cross-section morphology of the coated pellets are meant. Data for the dissolution profile obtained by the pellets according to the present invention are shown in the examples. Furthermore, the process of the present invention is also suitable for the use with coating suspensions free from ethanol.

The experiments were performed using a Pellegrini-type drum without perforation. For the purpose of the coating of pellets according to the present invention, any vessel having non-perforated wall (e.g. traditional copper pot, acid-resistant vessel having cone-cylinder-cone geometry) is also suitable.

Further details of the invention are disclosed in the following examples without limiting the the invention to said examples.

### Example 1

A bulk pellet mass is prepared on a laboratory scale. In the first step, a potassium chloride premix is prepared. For this purpose, potassium chloride of pharmaceutical grade containing 0.2 percent of weight colloidal silicon dioxide (Aerosil 200) is transformed into a powder using a pin mill (Alpine 160Z) wherein 90 percent by weight of the particles has a particle size less than 100 µm.

In the next step, seeding pellets are prepared. 255 g of potassium chloride premix are homogenized with 45 g of microcrystalline cellulose (Avicel PH 105) in a laboratory-scale centrifugal-fluidization granulating device (disc diameter 185 mm, volume of container approx. 4 dm³). Subsequently pellet particles were prepared by treating the homogenized powder with the solution prepared from 2.10 g silicone oil emulsion (containing 39 percent by weight of silicone oil) and 136.96 g of purified water. Wet particles are dried in the same apparatus. Thus pellets having particle size less than 0.8 mm are obtained as main fraction, which are used as seeding pellets in the subsequent steps.

In the next stage, pellets are prepared using seeding pellets. 255 g of potassium chloride premix, 45 g of microcrystalline cellulose (Avicel PH105) and 54.55 g of seeding pellets prepared according to the above process are homogenized in a laboratory-scale centrifugal-fluidization granulating apparatus (disc diameter 185 mm, volume of container approx. 4 dm³). The proportion of the seeding pellets in the thus obtained mixture is approximately 17 percent by weight. Pellets are prepared by treating the powder mixture with a granulating liquid prepared from 2.33 g silicon oil emulsion (silicon oil content of 39 percent by weight) and 152.18 g of purified water. Wet particles are dried in the same apparatus and sieved in a 0.8-1.6 mm mesh sieve to obtain a product fraction of approx. 150-200 g having a particle size of 0.8 -1.6 mm.

### Example 2

A pellet mass is produced on a plant scale. In the first step, the potassium chloride premix is prepared. For this purpose, pharmaceutical grade potassium chloride containing 0.2 percent by weight of silicon dioxide (Aerosil 200) is powdered in a pin mill (Alpine 160 Z) to obtain a powder wherein 90 percent by weight of the particles has a particle size smaller than 100 µm.

In the second step, seeding pellets are prepared by homogenizing 95.00 kg of potassium chloride premix with 15.00 kg of microcrystalline cellulose (Avicel PH 105) in the pellet producing apparatus (Glatt GPCG 120). A granulating liquid is prepared by mixing 0.796 kg of silicone oil emulsion (39 percent by weight) and 40.50 kg of purified water. The liquid thus obtained is sprayed onto the solid phase using the rotofluidization equipment to produce particles. The particles thus obtained are spheronized (i.e. brough to spheric shape). The wet particles are dried in the same equipment. In the process, particles of the size smaller than 0.8 mm are produced as a main fraction, which are used as seeding pellets in the subsequent production.

In the subsequent step, pellets are produced using seeding pellets. 95.00 kg of potassium chloride premix, 15.00 kg of microcrystalline cellulose (Avicel PH 105) and 20 kg of seeding pellets, which have a particle size smaller than 0.8 mm and which are produced by the process described above, are homogenized. The proportion of the seeding pellets is 19 percent by weight. For the homogenization rotofluidization apparatus (Glatt GPCG 120) is used. The granulating liquid is prepared by mixing 0.854 kg of silicone oil emulsion (concentration 39 percent by weight) and 45.00 kg of purified water.

The granulating liquid thus obtained is sprayed onto the solid phase resulting in formation of pellets. The pellets thus obtained are spheronized (brought to spheric form). The wet particles are dried in the same apparatus and the dry product is sieved using a 0.8-1.6 mm mesh sieve. Thus approximately 110 - 115 kg of product fraction consisting of pellets having particle size between 0.8 - 1.6 mm are obtained.

The use of seeding pellets according to the present invention increased the yield of the product fraction significantly. In the rotofluidization process known according to the state of the art, 15-20 kg (13-18 percent by weight) of pellets having particle size under 0.8 mm, 70-75 kg (63-68 percent by weight) of product fraction having particle size between 0.8 and 1.6 mm and 15-20 kg (13-18 percent by weight) of large particles having particle size over 1.6 mm can be produced from a 110 kg-charge. When seeding pellets according to the present invention are applied, the starting materials are mixed with 20 kg of seeding pellets, therefore the charge size is 130 kg. In this case, 10-15 kg (7.5-11.5 percent by weight) of fine particles (particle size under 0.8 mm), 105-110 kg (80-85 percent by weight) of product fraction and 5-10 kg (4-8 percent by weight) of large particles (particle size over 1.6 mm) are obtained. The fraction of the fine pellets having particle size less than 0.8 mm can be utilized in a subsequent batch as seeding pellets, therefore the proportion of the waste is 4 to 8 percent by weight, which is significantly more advantageous than the amount of the waste in the process known from the prior art, which amounts to 26 to 36 percent by weight.

### Example 3

A pellet mass is produced on a plant scale according to the process of Example 2 with the modification that the seeding pellets (20 kg) are fed into the apparatus during the spraying of the granulating liquid instead of homogenizing with the potassium chloride premix prior to pelletization.

### Example 4

A pellet mass is produced on a plant scale according to the process of the Example 2 with the modification that the seeding pellets used in the process are not obtained from an individual production step intended specifically for the production thereof. Instead, pellets deriving from the previous production batches are sieved to obtain a fraction consisting of pellets having particle size under 0.8 mm. From this fraction, 20 kg are weighed and used as seeding pellets.

### Example 5

Coating suspension is produced on a laboratory scale. 0.22 g of Ariavit Indigo Carmine dye is dissolved in 22.87 g of purified water and added to the mixture of 343.05 g of distilled water and 106.80 g of ethanol (96 percent). 2.52 g of colloidal silicon dioxide (Aerosil 200) and 19.1 g of talc are suspended in the dye solution, and 33.16 g of silicone oil emulsion (Dimeticon E 1049; concentration 39 percent by weight) are added. 22.87 g of purified water are used for rinsing. After 15 minutes of stirring, 430.19 g of Eudragit NE 30D are added and rinsed with 107.15 g of purified water.

Preparation of the coating suspension is followed by the coating of the pellets. A 22-1 volume rolling bed apparatus is charged with 2.00 kg of pellets having particle size between 0.8-1.6 mm. The temperature and flow rate of the inlet warm air is set to 55 to 60 °C and 20 m³/h, respectively. The feed rate of the coating suspension is 7 g/min and vessel is rotated at a rotation speed of 15.5 rpm. The intermediate drying before the dosing of the hydrophilizating solution takes place until 33 to 35 °C seed temperature at a rotation speed of 10 rpm.

After intermediate drying, the hydrophilizating solution comprising the solution of 17.38 g of potassium chloride in 69.47 g of purified water is sprayed onto the coated pellet using a feed rate of 5.0 to 5.5 g/min. The final drying is performed at a rotation speed of 5 rpm until 37-41 °C seed temperature.

The dissolution profile of the coated pellet is carried out using the USP dissolution testing apparatus with a rotating basket using 900 ml of water temperated at 37 °C and a 750-mg pellet sample. Determination of the chloride ion is carried out by argentometry using potentiometric end-point detection. Results of the dissolution testing are the following: 1 hour: 4.75%; 2 hours: 22.62%, 4 hours: 50.25%, 6 hours: 73.21%.

### Example 6

Coated pellets are produced on a laboratory scale.

The coating suspension is prepared by dissolving 0.22 g of Ariavit Indigo Carmine dye in 365.92 g of purified water. In this solution, 2.52 g of colloidal silicon dioxide (Aerosil 200) and 19.10 g of talc are suspended and subsequently 33.16 g of silicone oil emulsion (Dimeticon E1049, 39 percent by weight concentration) is added. Subsequently, 22.87 g of purified water is used for rinsing.

After stirring for 15 minutes, 430.19 g Eudragit NE 30D are added to the suspension and rinsed with 107.15 g of purified water.

A rolling bed apparatus having 22 l capacity is charged with 2.00 kg of pellets having particle size of 0.8-1.6 mm. The temperature and flow rate of inlet warm air is set to 55-60 °C and 20 m³/h, respectively. The average feed rate of the suspension is 7 g/min and the drum is rotated at 15.5 rpm.

The intermediate drying before the dosing of the hydrophilizing solution is carried out until 33-35 °C seed temperature at the rotation speed of 10 rpm.

After intermediate drying, the hydrophilizing solution comprising 17.38 g of potassium chloride and 69.47 g of purified water) is sprayed onto the coated pellets using a feed rate of 5.0-5.5 g/minute. The final drying is carried out until 37-41 °C seed temperature using the rotation speed of 5 rpm.

The dissolution profile of the coated pellets is determined according to the procedure described in Example 5. The results of the dissolution testing are the following: 1 hour: 3,33%; 2 hours: 21,96%, 4 hours: 53,40%, 6 hours: 76,41%.

### Example 7

The process of the coating on a plant scale is carried out as follows. The coating suspension is prepared by dissolving 18.9 g of Ariavit Indigo Carmine dye in 40.00 kg of purified water and 0.52 kg of colloidal silicon dioxide (Aerosil 200) and 4.00 kg talc are suspended in the dye solution. Subsequently, 6.93 kg of 39 percent by weight silicone oil (Dimeticon E 1049) emulsion is added to the suspension obtained by the above procedure.

After 15 minutes stirring, 90.00 kg of Eudragit NE 30 D are added.

A rolling bed apparatus (IMA HP 400F) is charged with 363.33 kg of pellets having particle size of 0,8 - 1,6 mm and the coating suspension is sprayed onto said pellets. After loading the suspension, the solution of 7.26 kg potassium chloride in 25.00 kg of purified water (hydrophilizing solution) is sprayed onto the coated pellets. The thus obtained pellets are subjected to sieving using a 2-mm mesh size sieve as a security measure.

The dissolution profile of the coated pellets is determined according to the procedure described in Example 5. The results of the dissolution testing are the following: 1 hour: 7,93%; 2 hours: 27,93%, 4 hours: 60,46%, 6 hours: 81,42%.

### Example 8

The present example discloses a process for pellet coating on an industrial scale. The coating suspension is prepared by dissolving 1.62 g of Ariavit Indigo Carmine dye in 171.53 g of purified water. The thus obtained dye solution is added to a mixture of 2572.90 g of distilled water and 901.03 g of ethanol (96 %). In the solution thus obtained, 18.87 g of colloidal silicon dioxide (Aerosil 200) and 143.22 g of talc are suspended, and to the resulting suspension, 248.71 g of 39 percent by weight silicone oil emulsion (Dimeticon E1049) are added. For rinsing, 171.53 g of purified water are used.

After stirring the suspension for 15 minutes, 3226.42 g of Eudragit NE 30 D are added and rinsed with 803.60 g of purified water.

A pilot-plant scale, rolling-bed [rolling-drum] apparatus having the capacity of 85 liters is charged with 15.0 kg of pellets having particle size of 0.8-1.6 mm. The temperature and the flow rate of the inlet warm air are adjusted to 56-61 °C and 55 m³/h, respectively. The average feed rate of the suspension is 24 g/min. The rotation speed of the drum during the coating is 20 rpm. The intermediate drying before loading the hydrophilizing solution is carried out until the seed temperature of 33-35 °C at 15 rpm rotation speed. After loading the suspension, the hydrophilizing solution comprising the solution of 130.36 g of potassium chloride in 521.01 g of purified water is sprayed onto the pellets at a feed rate of 13 g/min. The final drying is carried out until a seed temperature of 37-41 °C is reached while using a rotation speed of 10 rpm.

The dissolution properties of the thus obtained pellets are determined according to the procedure described in Example 5. Results of the dissolution test are the following: 1 hour: 1,92%; 2 hours: 19,18%, 4 hours: 55,21%, 6 hours: 79,89%.

### Example 9

In this example, an industrial scale coating process is disclosed.

For the preparation of the coating suspension, 1.62 g of Ariavit Indigo Carmine dye are dissolved in 1372.21 g of purified water. In this dye solution, 18.87 g of colloidal silicon dioxide (Aerosil 200) and 143.22 g of talc are suspended. The thus obtained suspension is mixed with 248.71 g of 39 percent silicone oil emulsion (Dimeticon E 1049). For the rinsing, 85.76 g of purified water are used. After stirring for 15 minutes, the suspension is mixed with 3226.42 g of Eudragit NE 30 D and rinsed with 401.80 g of purified water.

A pilot-plant size rolling-bed (rolling drum) apparatus of 85 liters capacity is charged with 15.0 kg of pellets having particle size between 0.8 and 1.6 mm. The temperature and flow rate of the inlet warm air are set to 56-61 °C and 55 m³/h, respectively. The coating suspension is fed into the apparatus at a mass flow rate of 24 g/minute. The rotation speed of the drum is 20 rpm during the coating operation. Before the loading of the hydrophilizing solution, an intermediate drying is carried out until the seed temperature of 33-35 °C is reached. During the intermediate drying, the rotation speed of the drum is 15 rpm. After loading the coating suspension, a hydrophilizing solution comprising the solution of 130.36 g of potassium chloride in 521.01 g of purified water is sprayed onto the coated pellets. The hydrophilizing solution is sprayed at a flow rate of 13 g/min. The final drying is carried out until the seed temperature of 37-41 °C is obtained. The rotational speed of the apparatus during the final drying is 10 rpm (revolutions / minute).

The dissolution profile of the coated pellets is determined according to the procedure disclosed in Example 5. The results of the dissolution testing are the following: 1 hour: 3,39%; 2 hours: 23,04%, 4 hours: 59,33%, 6 hours: 82,79%.

## Claims

1. Process for the preparation of a pellet mass, which comprises homogenizing potassium, chloride as active ingredient or the premix containing thereof with auxiliary agents to obtain a starting powder mixture; spraying the thus obtained starting powder mixture with a granulating liquid during agitation; spheronizing, drying and fractionating the thus obtained particles, **characterized in that** the starting or partially pelletized powder mixture is admixed with seeding pellets of identical or essentially identical composition but smaller average particle size than the pellets to be produced.

2. Process according to claim 1, **characterized in that** the proportion of the seeding pellets admixed with the starting or partially pelletized powder mixture is between 5 and 25 percent by weight, preferably between 15 and 20 percent by weight.

3. Process according to claim 1 or claim 2, **characterized in that** the average particle size of the seeding pellets is smaller than the lower particle size limit of the product fraction.

4. Process according to claim 3, **characterized in that** the greatest particle size of the seeding pellets is smaller than the lower particle size limit of the product fraction.

5. A process according to any of claims 1 to 4, **characterized in that** the process is carried out in batch operation.

6. Process according to claim 5, **characterized in that** the process is a rotofluidization operation.

7. A process according to any of claims 1 to 6, **characterized in that** the seeding pellets are obtained as a fraction having smaller particle size than the particle size of the desired product from a production pellet batch.

8. A process according to any of claims 1 to 6, **characterized in that** the seeding pellets are produced in an independent step separated from the pelletization process.

9. Process according to any of claims 1 to 8 for the surface coating of pellets containing potassium chloride active ingredient, **characterized in that** the operation is carried out in a rolling-bed apparatus having non-perforated wall.

10. Process according to claim 9 **characterized in that** the apparatus having non-perforated wall is a Pellegrini-type vessel.

## Patentansprüche

1. Verfahren zur Herstellung einer Pelletmasse, umfassend das Homogenisieren von Kaliumchlorid als wirksamen Bestandteil oder der Kaliumchloridenthaltenden Vormischung mit Hilfsmitteln, um eine Ausgangspulvermischung zu erhalten; Versprühen der so erhaltenen Ausgangspulvermischung mit einer Granulierflüssigkeit unter Rühren; das Sphäronisieren, Trocknen und Fraktionieren der somit erhaltenen Teilchen, **dadurch gekennzeichnet, dass** der Ausgangspulvermischung oder teilweise pelletisierten Pulvermischung Keimpellets mit identischer oder im Wesentlichen identischer Zusammensetzung jedoch mit einer kleineren durchschnittlichen Teilchengröße als die Pellets, die hergestellt werden sollen, beigemischt werden.

2. Verfahren nach Anspruch 1, **dadurch gekennzeichnet, dass** der Anteil an Keimpellets, die der Ausgangspulvermischung oder teilweise pelletisierten Pulvermischung beigemischt sind, zwischen 5 und 25 Gewichtsprozent, vorzugsweise zwischen 15 und 20 Gewichtsprozent beträgt.

3. Verfahren nach Anspruch 1 oder 2, **dadurch gekennzeichnet, dass** die durchschnittliche Teilchengröße der Keimpellets kleiner ist als der untere Grenzwert der Teilchengröße der Produktfraktion.

4. Verfahren nach Anspruch 3, **dadurch gekennzeichnet, dass** die größte Teilchengröße der Keimpellets kleiner ist als der untere Grenzwert der Produktfraktion.

5. Verfahren nach einem der Ansprüche 1 bis 4, **dadurch gekennzeichnet, dass** das Verfahren im diskontinuierlichen Betrieb durchgeführt wird.

6. Verfahren nach Anspruch 5, **dadurch gekennzeichnet, dass** das Verfahren ein rotierendes Wirbelschichtverfahren ist.

7. Verfahren nach einem der Ansprüche 1 bis 6, **dadurch gekennzeichnet, dass** die Keimpellets als Fraktion mit einer kleineren Teilchengröße als die Teilchengröße des gewünschten Produkts aus einer Pelletchargenproduktion erhalten werden.

8. Verfahren nach einem der Ansprüche 1 bis 6, **dadurch gekennzeichnet, dass** die Keimpellets in einem von dem Pelletisierungsverfahren unabhängigen Schritt hergestellt werden.

9. Verfahren nach einem der Ansprüche 1 bis 8 zum Oberflächenbeschichten von Pellets, die Kaliumchlorid als wirksamen Bestandteil enthalten, **dadurch gekennzeichnet, dass** der Vorgang in einer Trommelbettvorrichtung mit einer nicht perforierten Wand ausgeführt wird.

10. Verfahren nach Anspruch 9, **dadurch gekennzeichnet, dass** die Vorrichtung mit der nicht perforierten Wand ein Behälter vom Pellegrinityp ist.

## Revendications

1. Procédé de préparation d'une masse de pellet, qui comprend homogénéiser du chlorure de potassium comme ingrédient actif ou le mélange préliminaire contenant celui-ci avec des agents auxiliaires pour obtenir un mélange de poudre de départ ; pulvériser le mélange de poudre de départ ainsi obtenu avec un liquide de granulation au cours de l'agitation ; sphéroniser, sécher et fractionner les particules ainsi obtenues, **caractérisé en ce que** le mélange de poudre de départ ou partiellement pelletisé est mélangé à des pellets d'ensemencement de composition identique ou essentiellement identique mais de taille particulaire moyenne inférieure aux pellets devant être produits.

2. Procédé selon la revendication 1, **caractérisé en ce que** la proportion des pellets d'ensemencement mélangés au mélange de poudre de départ ou partiellement pelletisé est comprise entre 5 et 25 pour cent en poids, de préférence entre 15 et 20 pour cent en poids.

3. Procédé selon la revendication 1 ou la revendication 2, **caractérisé en ce que** la taille particulaire moyenne des pellets d'ensemencement est inférieure à la limite de taille particulaire inférieure de la fraction de produit.

4. Procédé selon la revendication 3, **caractérisé en ce que** la taille particulaire la plus élevée des pellets d'ensemencement est inférieure à la limite de taille particulaire inférieure de la fraction de produit.

5. Procédé selon l'une quelconque des revendications 1 à 4, **caractérisé en ce que** le procédé est effectué en marche discontinue.

6. Procédé selon la revendication 5, **caractérisé en ce que** le procédé est une opération de rotofluidisation.

7. Procédé selon l'une quelconque des revendications 1 à 6, **caractérisé en ce que** les pellets d'ensemencement sont obtenus comme une fraction ayant une taille particulaire inférieure à la taille particulaire du produit souhaité provenant d'un lot de pellets de production.

8. Procédé selon l'une quelconque des revendications 1 à 6, **caractérisé en ce que** les pellets d'ensemencement sont produits dans une étape indépendante séparée du procédé de pelletisation.

9. Procédé selon l'une quelconque des revendications 1 à 8 pour l'enduction superficielle de pellets contenant un ingrédient actif de chlorure de potassium, **caractérisé en ce que** l'opération est effectuée dans un appareil à lit roulant ayant une paroi non perforée.

10. Procédé selon la revendication 9, **caractérisé en ce que** l'appareil ayant une paroi non perforée est une cuve de type Pellegrini.
